(19) Europäisches Patentamt
European Patent Office
Office européen des brevets

(11) Publication number: 0 404 532 A1

# (12) EUROPEAN PATENT APPLICATION

(21) Application number: 90306708.0

(22) Date of filing: 20.06.90

(51) Int. Cl.5: A61K 7/32, A61K 7/34, A61K 7/38

(30) Priority: 23.06.89 US 370558
24.10.89 US 426341

(43) Date of publication of application:
27.12.90 Bulletin 90/52

(84) Designated Contracting States:
AT BE CH DE DK ES FR GB GR IT LI LU NL SE

(71) Applicant: THE PROCTER & GAMBLE COMPANY
One Procter & Gamble Plaza
Cincinnati Ohio 45202(US)

(72) Inventor: Oh, Young Sik
5461 Sir Lancelot Lane
Fairfield, Ohio(US)

(74) Representative: Brooks, Maxim Courtney et al
Procter & Gamble (NTC) Limited Whitley Road Longbenton
Newcastle-upon-Tyne NE12 9TS(GB)

## (54) Antiperspirant compositions.

(57) Clear antiperspirant compositions, which exhibit clean and non-sticky feel upon application in addition to excellent antiperspirant efficacy, are disclosed. These compositions comprise an antiperspirant active which is solubilized in propylene glycol, glycerine, ethanol, and/or water, together with a cosolvent selected from specific poly (ethylene glycols), poly (propylene glycols), poly (ethylene glycol) (propylene glycols), alkyl ethoxylates/propoxylates, propylene glycol methyl esters, polyethoxylated/polypropoxylated diols, phenoxypropanols, phenoxyethanols, phenethyl alcohols, hydroxy dimethylsiloxanes, and mixtures thereof. Examples of useful cosolvents include dipropylene glycol, hexylene glycol, triethylene glycol, tripropylene glycol, butylene glycol, tetrapropylene glycol, pentapropylene glycol, dibutylene glycol, pentylene glycol, tetraethylene glycol, pentaethylene glycol, hexaethylene glycol, PPG-10 butanediol, the condensation products of lauryl alcohol with from about 4 to about 12 moles of ethylene oxide, propylene glycol methyl ethers, dipropylene glycol methyl ethers, tripropylene glycol methyl ethers, 6-hydroxy hexyl pentamethyldisiloxane, and 1,3-bis (3-hydroxypropyl) tetramethyldisiloxane. These compositions may be formulated as solutions, sprays, roll-ons, or gel sticks. The method of preventing and controlling perspiration wetness using these compositions is also disclosed.

EP 0 404 532 A1

Xerox Copy Centre

# ANTIPERSPIRANT COMPOSITIONS

This application is a continuation-in-part of U.S. patent application Serial No. 370,558, Oh, filed June 23, 1989.

## Technical Field

The present invention relates to antiperspirant compositions, which may be formulated as solutions (e.g., sprays or roll-ons) or gel sticks, providing the user with excellent antiperspirant efficacy, as well as clean and non-sticky aesthetics when applied to the skin.

## Background of the Invention

Antiperspirant compositions have become a part of many people's personal care and grooming regimen. The formulation of such compositions, however, generally is an exercise in efficacy and aesthetic trade-offs, with the resulting composition being acceptable overall, but not outstanding. For example, the formulation of a composition to give it good usage aesthetic properties (e.g., minimized residue on the skin, and/or a clean, dry and non-sticky feel) often compromises antiperspirant efficacy. It is also difficult to maximize usage aesthetics and efficacy, while creating a product with good physical properties (e.g., a gel stick which is clear and has an appropriate hardness). One approach which has been taken to at least partially address this trade-off issue involves the use of antiperspirant actives solubilized in propylene glycol, water or ethanol. Unfortunately, these products frequently have compromised antiperspirant efficacy or feel wet and sticky when applied to the skin.

A variety of publications disclose antiperspirant actives which are soluble in non-aqueous solvents, typically monohydric alcohols (e.g., ethanol). U.S. Patent 3,873,686, Beekman, issued March 25, 1975, (incorporated herein by reference) provides a useful summary of work in this area. The following publications relate to antiperspirant actives soluble in non-aqueous solvents and/or processes for making such actives: U.S. Patent 3,359,169, Slater et al., issued December 19, 1967; U.S. Patent 3,420,932, Jones et al., issued January 7, 1969; U.S. Patent 3,507,896, Jones et al., issued April 21, 1970; U.S. Patent 3,523,130, Jones et al., issued August 4, 1970; U.S. Patent 3,555,146, Jones et al., issued January 12, 1971; U.S. Patent 3,876,758, Beekman, issued April 8, 1975; Great Britain Patent Specifications 1,159,685 and 1,159,686, both published July 30, 1969, Armour Pharmaceutical Company; and European Published Patent Application 7,191, published January 23, 1980, Unilever Limited.

U.S. Patent 2,890,987, Hilfer, issued June 16, 1959, describes the preparation of relatively dilute solutions of astringent aluminum compounds in propylene glycol having relatively high water content. That these solutions have such characteristics (i.e., dilute actives and high water content) results from the method of preparation used wherein a hot aqueous solution of the astringent aluminum compound (maximum solubility in water typically less than about 50%) is mixed with hot propylene glycol. Also of interest is U.S. Patent 4,137,306, Rubino et al., issued January 30, 1979, which discusses the Hilfer U.S. Patent 2,890,987 and describes anhydrous propylene glycol solutions of alcohol-soluble astringent basic aluminum compounds.

European Published Patent Application 284,765, American Cyanamid Company, published October 5, 1988, teaches that the use of dipropylene glycol in soap-based gel cosmetic (e.g., deodorant) sticks provides good product and usage aesthetics. This disclosure is in contrast to the present invention, the compositions of which contain an antiperspirant active and are substantially free of soap-based gelling agents.

U.S. Patent 2,823,169, Brown, issued February 11, 1958, describes the formation of aluminum chlorine alcoholate complexes which may be used in antiperspirant products. Low levels of compounds, including dipropylene glycol, may be used as softeners in these products. U.S. Patent 3,287,223, Theile et al., issued November 22, 1966, describes liquid antiperspirant compositions which are rendered non-sticky by the addition of a specific saturated cyclic $C_{18}$-$C_{20}$ alcohol. The exemplified composition includes low levels of dipropylene glycol and very high levels of ethanol. U.S. Patent 4,062,938, Gary et al., issued December 13, 1977, describes aerosol antiperspirant compositions including aluminum hydroxybromide, ethanol, and a

polyhydric alcohol which may be dipropylene glycol. U.S. Patent 4,346,079, Roehl, issued August 24, 1982, teaches gel antiperspirant sticks containing dibenzyl sorbitol as a gelling agent and up to 10% of a propyleneethylene glycol polycondensate. Ethanol, dipropylene glycol and hexylene glycol are among the alcohols taught to be useful in these compositions. In contrast to these various compositions, lu the antiperspirant compositions of the present invention utilize solubilized antiperspirant actives together with relatively high levels of specific cosolvents, such as hexylene glycol, the condensation products of lauryl alcohol with from about 4 to about 12 moles of ethylene oxide, or dipropylene glycol, and, at most, relatively low levels of ethanol, to provide a clear product which has clean and non-sticky aesthetics together with excellent antiperspirant efficacy. It is the ability to obtain these benefits in a composition containing an antiperspirant active which forms the heart of the present invention.

It is an object of the present invention to provide a clear antiperspirant composition which, in addition to excellent antiperspirant efficacy, feels clean and not sticky when applied to the skin.

## Summary of the Invention

The present invention relates to clear antiperspirant compositions, substantially free of soap-based gelling agents, comprising:

(a) from about 5% to about 35% of an antiperspirant active;

(b) from about 5% to about 35% of a solvent used to solubilize the antiperspirant active - these solvents are selected from propylene glycol, glycerine, water, ethanol, and mixtures thereof (preferably propylene glycol); and

(c) from about 10% to about 80% of a cosolvent selected from butylene glycol, dibutylene glycol, pentylene glycol, hexylene glycol, poly (ethylene glycols) which are liquid and have a viscosity of no greater than about 100 cps at room temperature, poly (propylene glycols) which are liquid and have a viscosity of no greater than about 100 cps at room temperature, poly (ethylene glycol) (propylene glycols) which are liquid and have a viscosity of no greater than about 100 cps at room temperature, $C_6$-$C_{20}$ alkyl alkoxylates containing from about 2 to about 18 moles of ethylene oxide and/or propylene oxide, ethoxylated or propoxylated phenethyl alcohols, ethoxylated or propoxylated phenoxyethanol, ethoxylated or propoxylated phenoxypropanol, propylene glycol methyl ethers, dipropylene glycol methyl ethers, tripropylene glycol methyl ethers, $C_4$-$C_{22}$ diols containing from about 2 to about 18 moles of ethylene oxide and/or propylene oxide, hydroxy dimethylsiloxanes having the formula

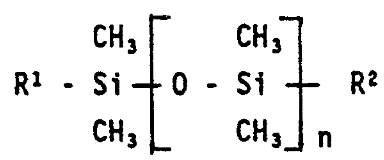

wherein n is from about 1 to about 4, and $R^1$ and $R^2$ are each independently selected from straight or branched-chain $C_1$-$C_{10}$ hydroxyalkyl groups; and mixtures thereof (preferably dipropylene glycol, hexylene glycol, PPG-10 butanediol, the condensation products of lauryl alcohol with from about 4 to about 12 moles of ethylene oxide, propylene glycol methyl ethers, dipropylene glycol methyl ethers, tripropylene glycol methyl ethers, 6-hydroxy hexyl pentamethyldisiloxane, and/or 1,3-bis(3-hydroxypropyl) tetramethyldisiloxane).

Compositions may be formulated as clear gel sticks which are substantially free of soap-based gelling agents and additionally comprise from about 1% to about 15% of a benzylidene alcohol gelling agent, such as dibenzylidene monosorbitol acetal.

## Detiled Description of the Invention

The antiperspirant compositions of the present invention include an antiperspirant active solubilized in propylene glycol, glycerine, ethanol and/or water, together with a specifically-selected cosolvent. The required, as well as the optional, components of the present invention are described in detail below. Of

course, all components used herein should be safe for application to skin at typical antiperspirant usage levels.

All percentages and ratios used herein are by weight unless otherwise indicated.

## Antiperspirant Material

The present compositions contain from about 5% to about 35%, preferably from about 10% to about 30%, by weight of an antiperspirant agent. These weight percentages are calculated on an anhydrous metal salt basis (exclusive of glycine, the salts of glycine, or other complexing agents). The antiperspirant agents are generally found in particulate form.

Any aluminum astringent antiperspirant salt or aluminum and/or zirconium astringent complex can be employed herein. Salts useful as astringent antiperspirant salts or as components of astringent complexes include aluminum halides, aluminum hydroxyhalides, zirconyl oxyhalides, zirconyl hydroxyhalides, and mixtures of these salt materials.

Aluminum salts of this type include aluminum chloride and the aluminum hydroxyhalides having the general formula $Al_2(OH)_xQ_y.XH_2O$ where Q is chloride, bromide or iodide (preferably chloride); where x is from about 2 to about 5, and $x+y$ = about 6, and x and y do not need to be integers; and where X is from about 1 to about 6. Aluminum salts of this type can be prepared in the manner described more fully in U.S. Patent 3,887,692, Gilman, issued June 3, 1975, and U.S. Patent 3,904,741, Jones and Rubino, issued September 9, 1975, incorporated herein by reference.

The zirconium compounds which are useful in the present invention include both the zirconium oxy salts and zirconium hydroxy salts, also referred to as the zirconyl salts and zirconyl hydroxy salts. These compounds may be represented by the following general empirical formula.

$ZrO(OH)_{2-nz}B_z$

wherein z may vary from about 0.9 to about 2 and need not be an integer; n is the valence of B; 2-nz is greater than or equal to 0; and B may be selected from the group consisting of halides (preferably chloride), nitrate, sulfamate, sulfate, and mixtures thereof. Although only zirconium compounds are exemplified in this specification, it will be understood that other Group IVB metal compounds, including hafnium, could be used in the present invention.

As with the basic aluminum compounds, it will be understood that the above formula is greatly simplified and is intended to represent and include compounds having coordinated and/or bound water in various quantities, as well as polymers, mixtures and complexes of the above. As will be seen from the above formula, the zirconium hydroxy salts actually represent a range of compounds having various amounts of the hydroxy group, varying from about 1.1 to only slightly greater than 0 groups per molecule.

Several types of antiperspirant complexes utilizing the above antiperspirant salts are known in the art. For example, U.S. Patent 3,792,068, Luedders et al., issued February 12, 1974, discloses complexes of aluminum, zirconium and amino acids such as glycines. Complexes such as those disclosed in the Luedders et al. patent and other similar complexes are commonly known as ZAG. ZAG complexes are chemically analyzable for the presence of aluminum, zirconium and chlorine. ZAG complexes useful herein are identified by the specification of both the molar ratio of aluminum to zirconium (hereinafter "Al:Zr" ratio) and the molar ratio of total metal to chlorine (hereinafter "Metal:Cl" ratio). ZAG complexes useful herein have an Al:Zr ratio of from about 1.67 to about 12.5 and a Metal:Cl ratio of from about 0.73 to about 1.93.

Preferred ZAG complexes are formed by

(A) co-dissolving in water

(1) one part $Al_2(OH)_{6-m}Q_m$, wherein Q is an anion selected from the group consisting of chloride, bromide and iodide; and m is from about 0.8 to about 2.0;

(2) x parts $ZrO(OH)_{2-a}Q_a.nH_2O$, where Q is chloride, bromide or iodide; a is from about 1 to about 2; n is from about 1 to about 8; and x is from about 0.16 to about 1.2;

(3) p parts neutral amino acid selected from the group consisting of glycine, d1-tryptophane, d1-$\beta$-phenylalanine, d1-valine, d1-methionine and $\beta$-alanine, and where p is from about 0.06 to about 0.53;

(B) co-drying the resultant mixture to a friable solid; and

(C) reducing the resultant dried inorganic-organic antiperspirant complex to particulate form.

A preferred aluminum compound for preparation of such ZAG type complexes is aluminum chlorhydroxide of the empirical formula $Al_2(OH)_5Cl.2H_2O$. Preferred zirconium compounds for preparation of such ZAG-type complexes are zirconyl hydroxychloride having the empirical formula $ZrO(OH)Cl.3H_2O$ and the zirconyl hydroxyhalides of the empirical formula $ZrO(OH)_{2-a}Cl_2.nH_2O$ wherein a is from about 1.5 to about 1.87, and n is from about 1 to about 7. The preferred amino acid for preparing such ZAG-type

complexes is glycine of the formula $CH_2(HN_2)COOH$. Salts of such amino acids can also be employed in the antiperspirant complexes. See U.S. Patent 4,017,599, Rubino, issued April 12, 1977, incorporated herein by reference.

A wide variety of other types of antiperspirant complexes are also known in the art. For example, U.S. Patent 3,903,258, Siegal, issued September 2, 1975, discloses a zirconium aluminum complex prepared by reacting zirconyl chloride with aluminum hydroxide and aluminum chlorhydroxide. U.S. Patent 3,979,510, Rubino, issued September 7, 1976, discloses an antiperspirant complex formed from certain aluminum compounds, certain zirconium compounds, and certain complex aluminum buffers. U.S. Patent 3,981,896, issued September 21, 1976, discloses an antiperspirant complex prepared from an aluminum polyol compound, a zirconium compound and an organic buffer. U.S. Patent 3,970,748, Mecca, issued July 20, 1976, discloses an aluminum chlorhydroxy glycinate complex of the approximate general formula $[Al_2(OH)_4Cl][H_2CNH_2COOH]$. All of these patents are incorporated herein by reference.

Of all the above types of antiperspirant actives, preferred compounds include the 5/6 basic aluminum salts of the empirical formula $Al_2(OH)_5Cl.2H_2O$; mixtures of $AlCl_3.6H_2O$ and $Al_2(OH)_5Cl.2H_2O$ with aluminum chloride to aluminum hydroxychloride weight ratios of up to about 0.5; ZAG type complexes wherein the zirconium salt is $ZrO(OH)Cl.3H_2O$, the aluminum salt is $Al_2(OH)_5Cl.2H_2O$ or the aforementioned mixtures of $AlCl_3.6H_2O$ and $Al_2(OH)_5Cl.2H_2O$ wherein the total metal to chloride molar ratio in the complex is less than about 1.25 and the Al:Zr molar ratio is about 3.3, and the amino acid is glycine; and ZAG-type complexes wherein the zirconium salt is $ZrO(OH)_{2-a}Cl_a.nH_2O$ wherein a is from about 1.5 to about 1.87 and n is from about 1 to about 7, the aluminum salt is $Al_2(OH)_5Cl.2H_2O$, and the amino acid is glycine.

The most preferred antiperspirant actives useful in the compositions of the present invention are antiperspirant actives with enhanced efficacy due to improved molecular distribution. Aluminum chlorhydroxide salts, zirconyl hydroxychloride salts, and mixtures thereof having improved molecular distributions are known, having been disclosed, for example, in the following documents, all incorporated by reference herein: U.S. Patent 4,359,456, Gosling et al., issued November 16, 1982; European Published Patent Application 6,739, Unilever Limited, published January 9, 1980; European Published Patent Application 183,171, Armour Pharmaceutical Company, published June 4, 1986; British Patent Specification 2,048,229, The Gillette Company, published December 10, 1980; European Published Patent Application 191,628, Unilever PLC, published August 20, 1986; and British Patent Specification 2,144,992, The Gillette Company, published March 20, 1985.

The improved molecular distribution is determined by the known analysis method called gel permeation chromatography. This analysis method is described, for example, in several of the above-incorporated patent specifications as well as in European Published Patent Application 7,191, Unilever Limited, published January 23, 1980, the disclosures of which are incorporated herein. It is preferred for purposes of the present invention that the antiperspirant actives utilized have enhanced efficacy due to improved molecular distribution with a ratio of peak 4 to peak 3 greater than about 0.1:1 as determined by gel permeation chromatography. This ratio, as is recognized by one skilled in the art, relates to the relative area under these two peaks as measured by the gel permeation chromatography analysis method.

## Solvent

The antiperspirant active is solubilized in propylene glycol, water, glycerine (1,2,3-trihydroxypropane), or mixtures thereof (e.g., propylene glycol/ethanol mixtures or glycerine/ethanol mixtures). As used herein, the term "solubilized" means that the antiperspirant active is dissolved in and/or colloidally dispersed (sub-micron particle size) in the solvent to give a transparent or semi-transparent liquid. Typically, the transparency of the liquid is such that more than 50% (preferably more than 75%) of 500 nm light is transmitted through the liquid as measured by a standard UV-visible absorption instrument. Propylene glycol is preferred for use herein, particularly when improved efficacy antiperspirant actives are used. The use of water to solubilize these actives can lead to a loss of efficacy. Further, when used, the level of ethanol should be limited to no greater than about 25%, preferably no greater than about 20%, most preferably no greater than about 15%, of the compositions in order to achieve the full aesthetic benefits offered by the present invention.

This solvent is present at from about 5% to about 35%, preferably from about 10% to about 30%, of the compositions of the present invention. Preferred compositions have weight ratios of antiperspirant active to solvent of from about 1:4 to about 1:0.8, preferably about 1:1.

Preferred solubilized antiperspirant actives for use in the present invention are described in U.S. Patent Application Serial No. 370,559, Smith and Ward, filed June 23, 1989, Solubilized Antiperspirant Actives and

Processes for Preparing the Same.

Cosolvent

The compositions of the present invention also comprise from about 10% to about 80%, preferably from about 20% to about 80%, most preferably from about 20% to about 65%, of particular cosolvents.

The cosolvents useful in the present invention have the following properties:

(1) they must be compatible with the material used to solubilize the antiperspirant active (propylene glycol, glycerine, ethanol, and/or water);

(2) they must be relatively non-volatile (boiling point at least about 120°C, preferably at least about 150°C);

(3) they should be in liquid form and not be too viscous (≤100 cps, preferably ≤75 cps) at room temperature;

(4) they should exhibit minimized penetration through the skin; and

(5) they should not cause the active to separate out of the final product.

Cosolvents which satisfy these criteria include butylene glycol; dibutylene glycol; pentylene glycol; hexylene glycol; poly (ethylene glycols) which are liquid and have a viscosity of no greater than about 100 cps at room temperature (e.g.. triethylene glycol; tetraethylene glycol, pentaethylene glycol, hexaethylene glycol), poly (propylene glycols) which are liquid and have a viscosity of no greater than about 100 cps at room temperature (e.g., dipropylene glycol, tripropylene glycol, tetrapropylene glycol, pentapropylene glycol); poly (ethylene glycol) (propylene glycols) which are liquid and have a viscosity of no greater than 100 cps at room temperature; alkyl alkoxylates which are liquid and have a viscosity of no greater than 100 cps at room temperature (e.g., $C_6$-$C_{20}$ alkyl alkoxylates containing from about 2 to about 18 moles of ethylene oxide and/or propylene oxide, as long as they satisfy the stated liquid/viscosity requirements, such as the condensation product of lauryl alcohol with 4, 7 or 10 moles of ethylene oxide); ethoxylated or propoxylated phenethyl alcohols; ethoxylated or propoxylated phenoxyethanol; ethoxylated or propoxylated phenoxypropanol; mono- and polypropylene glycol methyl ethers (e.g., propylene, dipropylene or tripropylene glycol methyl ethers); polypropoxylated or polyethoxylated (containing from about 2 to about 18 moles of ethylene oxide and/or propylene oxide) $C_4$-$C_{22}$ diols (e.g., PPG-10 butanediol) as long as they satisfy the stated liquid/viscosity requirements; hydroxy dimethylsiloxanes; and mixtures thereof.

The hydroxy dimethylsiloxanes useful herein have the formula

$$R^1 - \underset{CH_3}{\overset{CH_3}{Si}} - \left[ O - \underset{CH_3}{\overset{CH_3}{Si}} \right]_n - R^2$$

wherein n is from about 1 to about 4 (preferably 1 or 2, most preferably 1); and $R^1$ and $R^2$ are each independently selected from straight or branched-chain $C_1$-$C_{10}$ hydroxyalkyl groups. $R^1$ and $R^2$ may contain more than one hydroxyl group and, in fact, if $R^1$ or $R^2$ contains 6 or more carbon atoms, it is preferred that the moiety contains two or more hydroxyl groups. It is preferred that $R^1$ and $R^2$ be $C_1$-$C_6$ (most preferably $C_3$ or $C_4$) monohydroxy alkyl moieties. A preferred hydroxy dimethylsiloxane for use herein is 1,3-bis(3-hydroxypropyl) tetramethyldisiloxane, having the formula

$$HOCH_2CH_2CH_2\underset{CH_3}{\overset{CH_3}{Si}}--O--\underset{CH_3}{\overset{CH_3}{Si}}-CH_2CH_2CH_2OH$$

Another preferred hydroxy dimethylsiloxane for use herein is 6-hydroxy hexyl pentamethyldisiloxane.

Preferred cosolvents include dipropylene glycol, hexylene glycol, triethylene glycol, tripropylene glycol, butylene glycol, PPG-10 butanediol, the condensation products of $C_{10}$-$C_{14}$ alcohols (preferably $C_{12}$) with from about 4 to about 12 moles of ethylene oxide, propylene glycol methyl ether, dipropylene glycol methyl ether, tripropylene glycol methyl ether, 1,3-bis(3-hydroxypropyl) tetramethyldisiloxane, 6-hydroxy hexyl pentamethyldisiloxane, and mixtures thereof. Particularly preferred cosolvents are selected from dipropylene glycol, hexylene glycol, PPG-10 butanediol, the condensation products of $C_{10}$-$C_{14}$ alcohols (preferably $C_{12}$)

with from about 4 to about 12 moles of ethylene oxide, propylene glycol methyl ethers, dipropylene glycol methyl ethers, tripropylene glycol methyl ethers, 1,3-bis(3-hydroxypropyl) tetramethyldisiloxane, 6-hydroxy hexyl pentamethyldisiloxane, and mixtures thereof.

## Optional Components

The compositions of the present invention may be formulated as solutions, such as sprays or roll-ons, or clear gel sticks. Optional components utilized in these compositions will depend upon the particular physical and usage properties desired in the final product.

For liquid products, achieving the proper viscosity is very important. The addition of from about 0.1% to about 15%, preferably from about 1% to about 10%, propylene carbonate reduces the viscosity of the compositions to a desirable level. The choice of cosolvent also can provide particular viscosity characteristics. For example, propylene glycol methyl ethers and 1,3-bis(3-hydroxypropyl) tetramethyldisiloxane provide good viscosity characteristics for a liquid product. Other viscosity modifiers known in the art, such as dimethyl isosorbide, may also be used in the compositions of the present invention. When used, such viscosity modifiers are present at from about 1% to about 20% of the compositions.

Lower ($C_2$-$C_4$) monohydric alcohols, such as ethanol, may be used in the formulation of the compositions of the present invention. However, when such alcohols are used their levels should be limited to no greater than about 25%, preferably no greater than about 20%, most preferably no greater than about 15%, of the compositions since higher levels tend to make the compositions feel wet and cold upon application (both of which are undesirable aesthetic properties), and cause stinging when applied to the skin.

When the compositions of the present invention are formulated as clear gel sticks, they additionally contain from about 0.1% to about 15%, preferably from about 0.5% to about 5%, of a gelling agent, preferably a benzylidene alcohol gelling agent. The gelling agent should not be a soap, such as sodium stearate, since soaps tend to be incompatible with the antiperspirant actives. Thus, the compositions of the present invention must be substantially free of such soap-based gelling agents (i.e., they contain no more than about 0.5% of such soap-based gelling agents, if used at all).

Benzylidene alcohol gelling agents are disclosed in British Patent Specification 1,291,819, published October 4, 1972, incorporated herein by reference. The particular optimum gelling agent used will depend on the polarity of the solvent and cosolvent included in the composition. A preferred benzylidene alcohol gelling agent is dibenzylidene monosorbitol acetal (DBMSA). This material is commercially available, such as GELL-ALL-D (manufactured by New Japan Chemical Company Limited) and MILLITHIX 925 (manufactured by Millikin Chemical, Division of Millikin & Company). As will be appreciated by those skilled in the art, certain of the gel compositions containing benzylidene sorbitol may need to be buffered in order to provide effective and/or stable compositions. Such sticks may include a buffering agent (e.g., at a level of from about 0.1% to about 1%) so as to maintain a pH in the composition which is optimum for antiperspirant efficacy as well as stability of the gelling agent. Such buffering agents (e.g., diethanolamine, triethanolamine, glycine and ditallowmethylamine) are described in U.S. Patent 4,154,816, Roehl et al., issued May 15, 1975; U.S. Patent 4,346,079, Roehl et al., issued August 24, 1982; and U.S. Patent 4,518,582, Schamper et al., issued May 21, 1985, all of which are incorporated herein by reference.

The specific materials and their levels to be included in gel sticks of the present invention are selected to produce a stick of desired hardness to maintain dimensional stability while depositing a suitable amount of active material on the skin during normal use. Hardness of sticks can be determined in a variety of methods including American Society for Testing Materials (ASTM) Method D-5. This method involves the use of a needle or polished cone of particular weight and dimension which is allowed to travel downward through the stick material for a predetermined period of time. The distance traveled by the needle or cone is a relative measure of stick hardness. Utilizing Method D-5, with a penetration cone (Model H1310, sold by Humboldt Mfg. Co.) weighing 2.52 grams, and a Precision Model 14AN-8 Penetrometer (GCA Corp.), the gel sticks of the present invention preferably have a penetration value of from about 80 to about 180 mm over a period of 5 seconds.

Additional antiperspirant carriers useful in the present invention are well-known, and the selection of antiperspirant carriers and their appropriate usage levels for use in the antiperspirant compositions of the present invention can be readily made by one skilled in the art. Antiperspirant carriers include hardeners, strengtheners, solidifying agents, chelating agents, emollients, colorants, perfumes, emulsifiers, propellants, preservatives, and fillers. Antiperspirant carrier components are described more fully in the following publications, the disclosures of which are incorporated by reference herein: Plechner, "Antiperspirants and Deodorants, *Cosmetics, Science and Technology, 2*, pages 373-416 (Balsam and Sagarin, editors; 1972);

Fox, "Gel and Sticks Review and Update, *Cosmetics and Toiletries*, *99*, pages 19-52 (1984); Geria, "Formulation of Stick Antiperspirants and Deodorants", *Cosmetics and Toiletries*, *99*, pages 55-99 (1984); and Gels and Sticks Formulary, *Cosmetics and Toiletries*, *99*, pages 77-87 (1984). These antiperspirant carriers may also include other (i.e., non-antiperspirant) deodorant actives, such as anti-microbials or bacteriocides.

Examples of emollients which may be used in the compositions of the present invention include $C_{12}$-$C_{15}$ alkyl benzoates; $C_{12}$-$C_{15}$ alkyl lactates; fatty alcohols; esters, diesters or tetraesters of $C_2$-$C_{20}$ alcohols and $C_6$-$C_{16}$ fatty acids (e.g., diisopropyl sebacate, diisopropyl adipate (especially preferred), lauryl lactate, myristyl myristate, and pentaerythrityl tetrapelargonate); vola tile silicones (e.g., cyclic or linear polydimethylsiloxanes); polypropylene glycol ("PPG") ethers of $C_4$-$C_{22}$ (preferably $C_{10}$-$C_{20}$) fatty alcohols; and mixtures thereof. Cyclic polydimethylsiloxanes useful in the present compositions contain from about 3 to about 7 silicon atoms, and linear polydimethylslloxanes contain from about 3 to about 9 silicon atoms. A description of volatile silicones is found in Todd and Byers, "Volatile Silicone Fluids for Cosmetics", *Cosmetics and Toiletries*, 91:27-32 (1976), incorporated herein by reference. The volatile silicones and esters, diesters and tetraesters of $C_2$-$C_{20}$ alcohols and $C_6$-$C_{16}$ fatty acids, described above, are preferred emollients. When used, emollients comprise from about 2% to about 40% of the compositions of the present invention.

Solidifying agents which may be used in gel stick antiperspirant compositions of the present invention are waxy materials typically incorporated at a level of from about 1% to about 35%, preferably from about 10% to about 30%. Among such waxy materials useful herein are the high melting point waxes having a melting point of from about 65° C to about 102° C, low melting point waxes having a melting point of from about 37° C to about 75° C, and preferably mixtures thereof. High melting point waxes include beeswax, spermaceti, carnauba, baysberry, candelilla, montan, ozokerite, ceresin, paraffin, hydrogenated castor oil (castor wax), synthetic waxes such as Fisher-Tropsch waxes, microcrystalline waxes, and mixtures thereof. Castor wax is a preferred high-melting point wax useful herein. Low melting point waxes include fatty acids, fatty alcohols, fatty acid esters, and fatty acid amides, having fatty chains of from about 1 to about 30, preferably from about 12·to about 18, carbon atoms, and mixtures thereof. Preferred low melting point waxes include cetyl alcohol, palmitic acid, cetyl stearate, cetyl palmitate, and mixtures thereof.

Non-volatile paraffinic hydrocarbon fluids, present at a level of from about 3% to about 20% of the composition, may be included to reduce the residue left on the skin after application. Examples of such materials include mineral oils and $C_{16}$-$C_{68}$ highly branched aliphatic hydrocarbon liquids (such as Permethyls, marketed by Permethyl Corp., and Isopars, marketed by Exxon).

The compositions of the present invention, when formulated as sticks, may also contain a coupling agent. The term "coupling agent", as used herein, means any compound, composition, or combination thereof which acts to bring the polar and non-polar components of the present invention into a homogeneous gel stick composition. Preferably the coupling agent is selected from the group consisting of $C_6$-$C_{22}$ fatty alcohols, ethoxylated derivatives of $C_4$-$C_{22}$ fatty alcohols, propoxylated derivatives of $C_4$-$C_{22}$ fatty alcohols, and mixtures thereof as described, for example, in *Drug & Cosmetic Industry*, 138 (2), p. 40 (1986), the disclosures of which are incorporated herein by reference. More preferred coupling agents are ethoxylated derivatives of $C_{10}$-$C_{20}$ fatty alcohols, propoxylated derivatives of $C_{10}$-$C_{20}$ fatty alcohols, and mixtures thereof. The coupling agents to be used in the compositions of the present invention further most preferably have a Hydrophile-Lipophile Balance ("HLB) value within the range of from about 5 to about 18, preferably from about 8 to about 15. HLB is fully described, and values for various materials are provided, in the publication *The HLB System, A Time-Saving Guide to Emulsifier Selection* (published by ICI Americas Inc., Wilmington, DE; 1984), the disclosures of this publication being incorporated by reference herein.

More preferred coupling agents for use herein are polypropylene glycol ("PPG") ethers of $C_4$-$C_{22}$ - (preferably $C_{10}$-$C_{20}$) fatty alcohols. Examples of such materials are: PPG-4 myristyl ether, PPG-4 lauryl ether, PPG-10 cetyl ether, PPG-3 myristyl ether, and mixtures thereof. Additional examples are found in *CTFA Cosmetic Ingredient Dictionary*, Third Edition (Estrin et al., Editors; The Cosmetic, Toiletry and Fragrance Association, Inc., 1982), pages 252-26u and 494-500, the disclosures of which are incorporated herein by reference. Most preferred are PPG-10 cetyl ether (HLB about 14.5), PPG-3 myristyl ether (HLB about 9.8), and mixtures thereof.

The coupling agents, when used, typically comprise from about 1% to about 30%, preferably from about 5% to about 25%, and most preferably from about 15% to about 25%, of the compositions of the present invention.

Methods of Manufacture

The antiperspirant compositions of the present invention may be manufactured using methods known in the art.

In making liquid compositions, the antiperspirant active is solubilized in the solvent with mixing. In some instances heating may be required (e.g., to at least about 80° C) to achieve this solubilization (this is especially important when propylene glycol is the solvent). The remaining components are then added to the composition. In order to form a gel stick composition, the gelling agent and cosolvent are combined and heated to at least about 88° C. The solubilized antiperspirant active is then added and the mixture is poured into molds and cooled.

Method of Use

The antiperspirant compositions of the present invention are used in a conventional manner. Specifically, the compositions may be used to prevent and/or control perspiration wetness by topically applying, one or more times a day; an effective amount of the composition to areas of the body particularly prone to perspiration (e.g., the underarm (axillary) area).

The following non-limiting examples illustrate the compositions, methods of manufacture, and methods of use described in the present application.

Example I

The following is a liquid antiperspirant composition of the present invention. This composition may be used as a non-aerosol spray or roll-one

| Component | Weight % |
|---|---|
| Dipropylene glycol | 60 |
| Improved efficacy aluminum chlorhydroxide antiperspirant active (IACH) [1] | 15 |
| Propylene glycol | 15 |
| Ethanol | 8 |
| Perfume | 2 |

[1] Commercially available from Westwood Chemical Corporation.

The composition is made as follows. The propylene glycol is heated to 80° C. The IACH is then added and mixed to form a clear solution. This solution is cooled to ambient temperature and the remaining components are added and mixed together.

Example II

A liquid antiperspirant composition, which may be used as a non-aerosol spray, is as follows.

| Component | Weight % |
|---|---|
| 1,3-bis(3-hydroxypropyl)tetramethyldisiloxane | 60 |
| IACH | 15 |
| Propylene glycol | 15 |
| Ethanol | 8 |
| Perfume | 2 |

The composition is made as follows. The propylene glycol is heated to 80° C. The IACH is then added and mixed to form a clear solution. This solution is then cooled to ambient temperature and the remaining components are added and mixed together.

Example III

A clear gel stick antiperspirant composition of the present invention is as follows.

| Component | Weight % |
|---|---|
| Dipropylene glycol | 63 |
| IACH | 15 |
| Propylene glycol | 15 |
| Propylene carbonate | 2 |
| Millithix 925 (DBMSA) [1] | 3 |
| Perfume | 2 |

[1] Commercially available from Millikin Chemical.

The composition is made as follows. The solubilized IACH is prepared by mixing together and heating the powdered IACH and propylene glycol. The dipropylene glycol and propylene carbonate are combined and heated to about 50° C. The DBMSA is added and the mixture is heated to about 116-118° C forming a liquid mixture. The solubilized IACH active is heated to about 60° C. When the DBMSA mixture has cooled to 80-90° C, the solubilized IACH and perfume are added and mixed for about 2 minutes. The entire mixture is then poured into stick molds and cooled for about 1 hour.

Example IV

A clear gel stick antiperspirant composition of the present invention is as follows.

| Component | Weight % |
|---|---|
| IACH | 15 |
| Hexylene glycol | 20 |
| Propylene glycol | 15 |
| $C_{12}$-$C_{15}$ alkyl benzoate | 5 |
| Millithix 925 (DBMSA) | 3 |
| Dipropylene glycol | 34 |
| Propylene carbonate | 8 |

The solubilized IACH active is prepared by mixing together and heating the powdered IACH and propylene glycol. The hexylene glycol, dipropylene glycol, propylene carbonate, $C_{12}$-$C_{15}$ alkyl benzoate and propylene carbonate are combined and heated to about 50° C. The DBMSA is added and the mixture is heated to from about 100-105° C forming a liquid mixture. The solubilized IACH active is heated to about 60° C. When the DBMSA mixture has cooled to 80-90° C, the solubilized IACH active is added and mixed for about 2 minutes. The entire mixture is then poured into stick molds and allowed to stand for approximately one hour.

Example V

A liquid antiperspirant composition is as follows.

| Component | Weight % |
|---|---|
| IACH | 15 |
| Dipropylene glycol | 30 |
| Hexylene glycol | 24 |
| Water | 20 |
| Propylene carbonate | 10 |
| Perfume | 1 |

The composition is made as follows. The IACH is added to water and mixed to form a clear solution. The remaining components are added and mixed together.

## Example VI

A liquid antiperspirant composition, which may be used as a non-aerosol spray, is as follows.

| Component | Weight % |
|---|---|
| IACH | 10 |
| Laureth 4 [1] | 25 |
| D5 cyclomethicone [2] | 21 |
| Diisopropyl adipate | 16 |
| Ethanol | 15 |
| Propylene glycol | 10 |
| DRO water | 2 |
| Perfume | 1 |

[1] Condensation product of lauryl alcohol with 4 moles of ethylene oxide, commercially available from ICI Americas.
[2] A cyclic polydimethylsiloxane containing 5 carbons, commercially available from General Electric Silicones.

The composition is made as follows. The propylene glycol is heated to 80° C. The IACH is then added and mixed to form a clear solution. This solution is cooled to ambient temperature and the remaining components are added and mixed together.

## Example VII

A liquid antiperspirant composition, which may be used as a non-aerosol spray, is as follows.

| Component | Weight % |
|---|---|
| IACH | 15 |
| Laureth 4 | 24 |
| D5 cyclomethicone | 20 |
| Ethanol | 20 |
| PPG-3 myristyl ether | 10 |
| Propylene glycol | 8 |
| DRO water | 2 |
| Perfume | 1 |

The composition is made as follows. The propylene glycol is heated to 80° C. The IACH is then added and mixed to form a clear solution. The solution is cooled to ambient temperature and the remaining components are added and mixed together.

## Example VIII

A liquid antiperspirant composition, which may be used as a non-aerosol spray, is as follows.

| Component | Weight % |
|---|---|
| IACH[1] | 15 |
| Propylene glycol | 15 |
| Dipropylene glycol methyl ether | 15 |
| Tripropylene glycol methyl ether | 15 |
| Ethanol | 14 |
| Laureth 4 | 10 |
| Laureth 7[1] | 10 |
| Propylene glycol methyl ether | 5 |
| Perfume | 1 |

[1] Condensation product of lauryl alcohol with 7 moles of ethylene oxide, commercially available from ICI Americas.

The composition is made as follows. The propylene glycol is heated to 80° C. The IACH is then added and mixed to form a clear solution. This solution is cooled to ambient temperature and the remaining components are added and mixed together.

The antiperspirant compositions described in Examples I-VIII, when applied to the axillary area of the user, provide effective prevention and control of perspiration wetness. The compositions have excellent aesthetics and feel clean and not sticky when applied to the skin.

## Claims

1. An antiperspirant composition, substantially free of soap-based gelling agents, characterized in that it comprises:
(a) from 5% to 35% of an antiperspirant active;
(b) from 5% to 35%, preferably from 10% to 30%, of a solvent selected from propylene glycol, glycerine, ethanol, water, and mixtures thereof, within which the antiperspirant active is solubilized; and
(c) from 10% to 80%, preferably from 20% to 65%, of a cosolvent selected from butylene glycol, dibutylene glycol, pentylene glycol, hexylene glycol, poly (ethylene glycols) which are liquid and have a viscosity of no greater than about 100 cps at room temperature, poly (propylene glycols) which are liquid and have a viscosity of no greater than about 100 cps at room temperature, poly (ethylene glycol) (propylene glycols) which are liquid and have a viscosity of no greater than about 100 cps at room temperature, $C_6$-$C_{20}$ alkyl

alkoxylates containing from 2 to 18 moles of alkoxy groups selected from ethylene oxide, propylene oxide, and mixtures thereof which are liquid and have a viscosity of no greater than about 100 cps at room temperature, ethoxylated or propoxylated phenethyl alcohols, ethoxylated or propoxylated phenoxyethanol, ethoxylated or propoxylated phenoxypropanol, propylene glycol methyl ethers, dipropylene glycol methyl ethers, tripropylene glycol methyl ethers, $C_4$-$C_{22}$ diols containing from 2 to 18 moles ot alkoxy groups selected from ethylene oxide, propylene oxide, and mixtures thereof which are liquid and have a viscosity of no greater than about 100 cps at room temperature, hydroxydimethylsiloxanes having the formula:

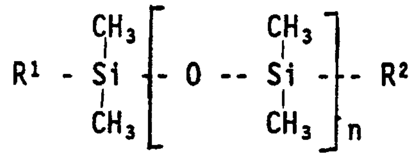

wherein n is from 1 to 4, and $R^1$ and $R^2$ are independently selected from straight or branched-chain $C_1$-$C_{10}$ hydroxyalkyl groups; and mixtures thereof;
such that when ethanol is used it comprises no more than about 25%, preferably no more than about 20%, of said antiperspirant composition.

2. An antiperspirant composition according to Claim 1 characterized in that the antiperspirant active is selected from aluminum chlorhydroxide salts, zirconyl hydroxychloride salts, and mixtures thereof, and preferably has enhanced efficacy due to improved molecular distribution with a ratio of peak 4 to peak 3 greater than about 0.1:1 as determined by gel permeation chromatography.

3. An antiperspirant composition according to Claim 1 or 2 characterized in that the solvent comprises propylene glycol.

4. An antiperspirant composition according to any of Claims 1-3 characterized in that the cosolvent is selected from dipropylene glycol, hexylene glycol, triethylene glycol, tripropylene glycol, butylene glycol, PPG-10 butanediol, condensation products of $C_{10}$-$C_{14}$ alcohols with from 4 to 12 moles of ethylene oxide, propylene glycol methyl ethers, dipropylene glycol methyl ethers, tripropylene glycol methyl ethers, 1,3-bis-(3-hydroxypropyl) tetramethyldisiloxane, 6-hydroxy hexyl pentamethyldisiloxane, and mixtures thereof.

5. An antiperspirant composition according to any of Claims 1-4 characterized in that the cosolvent is selected from dipropylene glycol, hexylene glycol, condensation products of $C_{10}$-$C_{14}$ alcohols with from 4 to 12 moles of ethylene oxide, propylene glycol methyl ethers, dipropylene glycol methyl ethers, tripropylene glycol methyl ethers, and mixtures thereof.

6. An antiperspirant composition according to any of Claims 1-5 characterized in that it additionally comprises from 0.1% to 15% propylene carbonate.

7. An antiperspirant composition according to any of Claims 1-6 characterized in that it additionally comprises from 2% to 40% of an emollient selected from volatile silicones; esters, diesters and tetraesters of $C_2$-$C_{20}$ alcohols and $C_6$-$C_{16}$ fatty acids; and mixtures thereof.

8. An antiperspirant composition according to any of Claims 1-7 in the form of a clear gel stick characterized in that it additionally comprises from 0.1% to 15% of a benzylidene alcohol gelling agent, preferably dibenzylidene monosorbital acetal.

9. A method for preventing and controlling perspiration wetness in humans characterized in that it comprises application to the underarm area of an effective amount of the antiperspirant composition according to any of Claims 1-8.

European Patent Office

# EUROPEAN SEARCH REPORT

Application number

EP 90306708.0

## DOCUMENTS CONSIDERED TO BE RELEVANT

| Category | Citation of document with indication, where appropriate, of relevant passages | Relevant to claim | CLASSIFICATION OF THE APPLICATION (Int Cl) |
|---|---|---|---|
| D,X | US - A - 4 518 582 (T.J.SCHAMPER et al.) * Column 2, lines 22-45; column 3, lines 23-41; examples * -- | 1-5,7-9 | A 61 K 7/32<br>A 61 K 7/34<br>A 61 K 7/38 |
| X | DE - A - 1 617 491 (H. LEIBIGER) * Pages 3,4; claim 1 * -- | 1,2,3,9 | |
| D,X | US - A - 4 346 079 (E.L. ROEHL) * Columns 3,4; examples * -- | 1,2,4,5,8,9 | |
| A | EP - A2 - 0 295 070 (THE PROCTER & GAMBLE CO.) * Claims * ---- | 1,2,3,7,9 | |

TECHNICAL FIELDS SEARCHED (Int Cl)

A 61 K 7/00

The present search report has been drawn up for all claims

| Place of search | Date of completion of the search | Examiner |
|---|---|---|
| VIENNA | 04-10-1990 | IRMLER |

CATEGORY OF CITED DOCUMENTS

X particularly relevant if taken alone
Y particularly relevant if combined with another document of the same category
A technological background
O non-written disclosure
P intermediate document

T theory or principle underlying the invention
E earlier patent document, but published on, or after the filing date
D document cited in the application
L document cited for other reasons
& member of the same patent family, corresponding document